# EUROPEAN PATENT APPLICATION

(11) **EP 4 628 021 A1**
(43) Date of publication of application: **08.10.2025**
(21) Application number: 25166553.5
(22) Date of filing: 27.03.2025
(51) Int. Cl.: A61B 6/10, A61B 6/00

(54) **MEDICAL APPARATUS**

(30) Priority: 01.04.2024 CN 202420663181 U
(71) Applicant: Siemens Healthineers AG, 91301 Forchheim (DE)
(72) Inventor: YANG, Qi Xin, Shanghai, 201206 (CN); WANG, Hong Sheng, Shanghai, 200124 (CN)
(74) Representative: HKW Intellectual Property PartG mbB

(57) **Abstract**

According to embodiments of the present disclosure, a medical apparatus is provided, comprising a main body and a support structure, the support structure being able to carry and move an object, the main body having a casing, wherein the medical apparatus further comprises: a sensing strip, affixed to a region on the casing that the object might touch, the sensing strip comprising a flexible thin-film pressure sensor and a soft elastic component, the flexible thin-film pressure sensor being tightly fitted between the soft elastic component and the casing, and outputting a pulse; an analog-to-digital converter, which receives a pulse from the flexible thin-film pressure sensor, and converts the pulse to a digital signal; and a controller, which receives the digital signal, and on this basis stops movement of the support structure. The medical apparatus of the present disclosure detects pressure via the flexible thin-film pressure sensor, can be flexibly arranged in a region where collision might occur, and is highly responsive with a quick response. When a collision occurs, emergency braking is triggered automatically, avoiding bodily harm.

## Description

In the present patent application, nouns and pronouns referring to people are not limited to a specific gender.

### TECHNICAL FIELD

The present disclosure relates to medical apparatus, in particular collision protection.

### BACKGROUND ART

As CT machines become ever more ubiquitous, medical accidents involving CT happen from time to time. One type of accident is where part of a patient's limb is not inside the range of the scanning aperture of the CT gantry as the patient table is moving, resulting in a collision between the limb and the gantry casing, in serious cases causing bodily injury to the patient.

Generally, a doctor will monitor the situation in the CT machine room in real time through a transparent glass window. If the doctor notices a collision, the doctor will press an emergency button to force the patient table to immediately stop moving, to avoid greater harm.

### SUMMARY OF THE DISCLOSURE

In view of this, the present disclosure proposes a medical apparatus.

According to embodiments of the present disclosure, a medical apparatus is provided, comprising a main body and a support structure, the support structure being able to carry and move an object to approach the main body or enter an open space of the main body, the main body having a casing, wherein the medical apparatus further comprises: a sensing strip, affixed to a region on the casing that the object might touch, the sensing strip comprising a flexible thin-film pressure sensor and a soft elastic component, the flexible thin-film pressure sensor being tightly fitted between the soft elastic component and the casing, and outputting a pulse upon detecting pressure applied thereto; an analog-to-digital converter, which receives a pulse from the flexible thin-film pressure sensor, and converts the pulse to a digital signal; and a controller, which receives the digital signal, and on this basis stops movement of the support structure.

In an embodiment, the flexible thin-film pressure sensor has a pressure-sensitive range of 20 g - 10 KG.

In an embodiment, the flexible thin-film pressure sensor has a response time of 1 ms.

In an embodiment, the sensing strip comprises a protective shell, the protective shell being tightly fitted to the soft elastic component and tightly fitted at two sides thereof to the casing.

In an embodiment, an entry/exit of the open space is a circular-arc-shaped surface with a circle center angle of 90°, and the sensing strip is affixed at a 45° position of at least one of the entry/exit.

In an embodiment, the medical apparatus further comprises a second sensing strip, affixed at a 30° position of the circular-arc-shaped surface.

In an embodiment, the sensing strip is a 270° circular ring.

In an embodiment, the medical apparatus is one of a CT machine, a magnetic resonance imaging apparatus, a PET apparatus, a PET-CT apparatus and a SPECT apparatus.

The medical apparatus of the present disclosure detects pressure via the flexible thin-film pressure sensor, can be flexibly arranged in a region where collision might occur, and is highly responsive with a quick response. When a collision occurs, emergency braking is triggered automatically, avoiding bodily harm.

### BRIEF DESCRIPTION OF THE DRAWINGS

Preferred embodiments of the present disclosure will be described in detail below with reference to the drawings, so that those of ordinary skill in the art have a clearer understanding of the above and other features and advantages of the present disclosure. In the drawings:
Fig. 1 is a schematic drawing of a medical apparatus according to an embodiment of the present disclosure.
Fig. 2 is a schematic sectional view of the medical apparatus of Fig. 1 along a perpendicular plane in which an aperture lies.
Fig. 3 is a schematic partial enlarged drawing of the section in Fig. 2.

The reference labels used in the abovementioned drawings are as follows:

| | | | |
|---|---|---|---|
| 100 | Medical apparatus | 110 | Sensing strip |
| 102 | Main body | 111 | Controller |
| 104 | Open space | 112 | Flexible thin-film pressure sensor |
| 106 | Support structure | 113 | Stop button |
| 108 | Casing | 114 | Soft elastic component |
| 109 | Analog-to-digital converter | 116 | Protective shell |

### DETAILED DESCRIPTION OF EMBODIMENTS

In order to clarify the object, technical solution and advantages of the present disclosure, the present disclosure is explained in further detail below by way of embodiments.

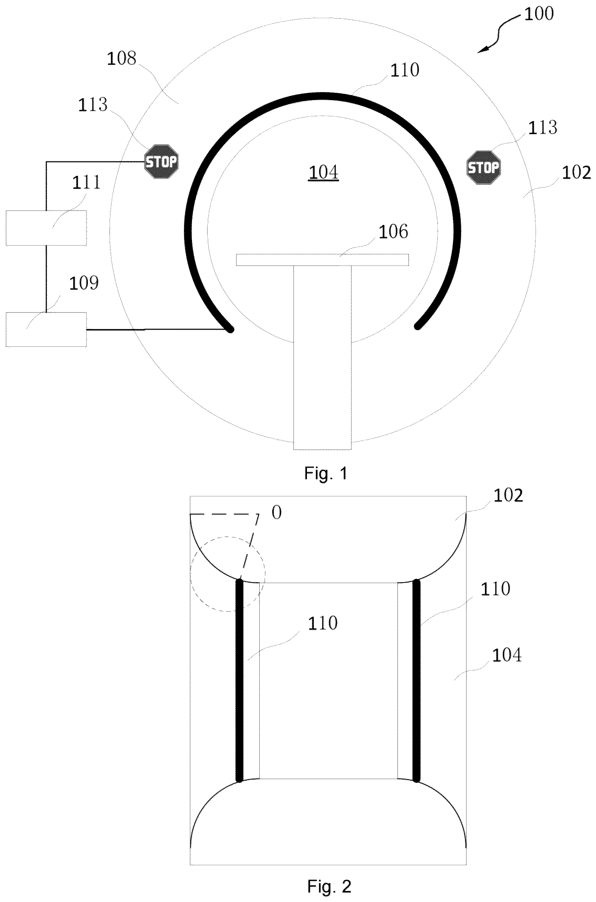

Fig. 1 is a schematic drawing of a medical apparatus 100 according to an embodiment of the present disclosure. The medical apparatus 100 may for example be one of a CT (computed tomography) machine, a magnetic resonance imaging apparatus, a PET (positron emission tomography) apparatus, a PET-CT apparatus, and a SPECT (single photon emission CT) apparatus. As shown in Fig. 1, the medical apparatus 100 comprises a main body 102 and a support structure 106. In this embodiment, the medical apparatus 100 is a CT machine, the main body 102 is a gantry, and the support structure 106 is a bed board. The support structure 106 is able to carry and move an object (e.g. a patient), to approach the main body 102 or enter an open space 104 of the main body 102, and the main body 102 has a casing 108. A stop button 113 is provided on the main body 102; when the stop button 113 is pressed, the support structure 106 will stop moving. Generally, stop buttons 113 are provided on both sides of the main body 102, to enable quick operation by a doctor at both sides. The medical apparatus 100 further comprises a sensing strip 110, an analog-to-digital converter 109 and a controller 111.

As shown in Fig. 1, in this embodiment, the sensing strip 110 is a 270° circular ring.

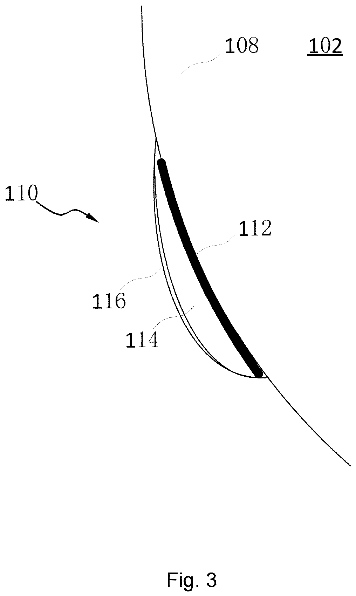

Fig. 2 is a schematic sectional view of the medical apparatus 100 of Fig. 1 along a perpendicular plane in which an aperture lies; Fig. 3 is a schematic partial enlarged drawing of the section in Fig. 2. As shown in Figs. 2 and 3, the sensing strip 110 is affixed to a region on the casing 108 which the object might touch. In this embodiment, the open space 104 is an aperture of the CT machine, and the region which the object might touch is an entry/exit of the aperture. The entry/exit of the open space 104 is a circular-arc-shaped surface with a circle center angle of 90°, and the sensing strip 110 is affixed at a 45° position of the entry/exit. In other embodiments, the sensing strip 110 may be affixed at the 45° position of only one of the entry/exit.

To increase coverage, in other embodiments, the medical apparatus 100 may also comprise a second sensing strip (not shown), which may be affixed at a 30° position of the circular-arc-shaped surface.

The sensing strip 110 comprises a flexible thin-film pressure sensor (FTFPS) 112 and a soft elastic component 114. In this embodiment, the soft elastic component 114 is a strongly elastic rubber body. The FTFPS 112 is tightly fitted between the soft elastic component 114 and the casing 108 of the main body 102, and outputs a pulse upon detecting pressure applied thereto.

Flexible thin-film pressure sensors come in a variety of forms and are easy to customize. A pulse will be triggered even when a finger is pressed on the sensor with a very small force. In this embodiment, the FTFPS used has a pressure-sensitive range of 20 g to 10 KG, with a response time of 1 ms. That is to say, as long as the pressure exceeds 20 g, the triggering mechanism will be activated within 1 ms. This can effectively prevent harm to the patient due to a collision.

The FTFPS has advantages such as being thin, easy to bend and sensitive to pressure, with a wide range of deployment and control and a single circuit, so is suitable for being affixed to the gantry casing to sense collisions.

In order to fit the gantry surface tightly, preferably, the flexible thin-film pressure sensor 112 must be custom-made in an arc shape, in both a length direction and a thickness direction.

The soft elastic component 114 can protect the sensor, and also helps to convert lateral forces to forces perpendicular to the sensor surface.

In this embodiment, the sensing strip 110 further comprises a protective shell 116 (of plastic material for example), the protective shell 116 being tightly fitted to the soft elastic component 114 and tightly fitted at two sides thereof to the casing 108. The protective shell 116 can make the sensing strip 110 as a whole more attractive in appearance, causes the flexible thin-film pressure sensor 112 and the soft elastic component 114 to be fitted more tightly to the casing 108 of the main body 102, and is also convenient to clean. The protective shell 116 is preferably smooth and tough.

The analog-to-digital converter 109 receives a pulse from the FTFPS 112, and converts the pulse to a digital signal.

The controller 111 receives the digital signal, and on this basis stops movement of the support structure 106. An interface end of the FTFPS 112 may be arranged at a side close to the controller 111. In this embodiment, a gantry static controller also serves as the controller 111, being connected to a central controller. The same is true for the opposite side.

The controller 111 can output a signal to the stop button 113, the signal being equivalent to the stop button 113 being pressed, so as to realize emergency braking of the support structure 106.

The medical apparatus of the present disclosure detects pressure via the flexible thin-film pressure sensor, can be flexibly arranged in a region where collision might occur, and is highly responsive with a quick response. When a collision occurs, emergency braking is triggered automatically, avoiding bodily harm.

The embodiments above are merely preferred embodiments of the present disclosure, which are not intended to limit it. Any amendments, equivalent substitutions or improvements etc. made within the spirit and principles of the present disclosure shall be included in the scope of protection thereof.

## Claims

1. A medical apparatus, comprising a main body (102) and a support structure (106), the support structure (106) being able to carry and move an object to approach the main body (102) or enter an open space (104) of the main body (102), the main body (102) having a casing (108), **characterized in that** the medical apparatus further comprises:
a sensing strip (110), affixed to a region on the casing (108) that the object might touch, the sensing strip (110) comprising a flexible thin-film pressure sensor (112) and a soft elastic component (114), the flexible thin-film pressure sensor (112) being tightly fitted between the soft elastic component (114) and the casing (108), and outputting a pulse upon detecting pressure applied thereto;
an analog-to-digital converter (109), which receives a pulse from the flexible thin-film pressure sensor (112), and converts the pulse to a digital signal;
a controller (111), which receives the digital signal, and on this basis stops movement of the support structure (106).

2. The medical apparatus as claimed in claim 1, **characterized in that** the flexible thin-film pressure sensor (112) has a pressure-sensitive range of 20 g - 10 KG.

3. The medical apparatus as claimed in claim 1, **characterized in that** the flexible thin-film pressure sensor (112) has a response time of 1 ms.

4. The medical apparatus as claimed in claim 1, **characterized in that** the sensing strip (110) comprises a protective shell (116), the protective shell (116) being tightly fitted to the soft elastic component (114) and tightly fitted at two sides thereof to the casing (108).

5. The medical apparatus as claimed in claim 1, **characterized in that** an entry/exit of the open space (104) is a circular-arc-shaped surface with a circle center angle of 90°, and the sensing strip (110) is affixed at a 45° position of at least one of the entry/exit.

6. The medical apparatus as claimed in claim 5, **characterized in that** it further comprises a second sensing strip, affixed at a 30° position of the circular-arc-shaped surface.

7. The medical apparatus as claimed in claim 5, **characterized in that** the sensing strip (110) is a 270° circular ring.

8. The medical apparatus as claimed in claim 1, **characterized in that** the medical apparatus is one of a CT machine, a magnetic resonance imaging apparatus, a PET apparatus, a PET-CT apparatus and a SPECT apparatus.
